# EUROPEAN PATENT SPECIFICATION

(11) **EP 3 148 526 B1**
(45) Date of publication and mention of the grant of the patent: **06.01.2021**
(21) Application number: 15799560.6
(22) Date of filing: 27.05.2015
(51) Int. Cl.: A61K 31/337, A61K 31/7068, A61K 31/35, A61K 31/357, A61K 31/282, A61K 31/427, A61K 31/555, A61K 31/704, A61K 33/24, A61K 45/06, A61P 35/00, A61P 15/08, C12Q 1/68, A61K 9/00

(54) **USE OF ERIBULIN IN THE TREATMENT OF CANCER**
VERWENDUNG VON ERIBULIN BEI DER BEHANDLUNG VON KREBS
UTILISATION DE L'ÉRIBULINE DANS LE TRAITEMENT DU CANCER

(30) Priority: 28.05.2014 US 201462003937 P
(43) Date of publication of application: 05.04.2017
(73) Proprietor: Eisai R&D Management Co., Ltd., Tokyo 112-8088 (JP)
(72) Inventor: LITTLEFIELD, Bruce A., Andover, MA 01810 (US); ASANO, Makoto, Tsukuba-shi Ibaraki 300-2635 (JP); MATSUI, Junji, Tsukuba Ibaraki 305-0035 (JP); OLIVO, Martin, Westwood, NJ 07675 (US); OZAWA, Yoichi, Tsukuba-shi Ibaraki 300-2635 (JP); YU, Yanke, San Diego, CA 92130-8606 (US)
(74) Representative: J A Kemp LLP
(86) International application number: PCT/US2015/032684
(87) International publication number: WO 2015/183961

(56) References cited:
- US-A1- 2006 104 984
- US-B1- 6 653 341
- Anonymous: "NCT01323530 on 2011_03_24: ClinicalTrials.gov Archive - A phase 1b/2 multicenter randomized open-label dose escalation and confirmation study of eribulin in combination with capecitabine", , 24 March 2011 (2011-03-24), XP055419867, Retrieved from the Internet: URL:https://clinicaltrials.gov/archive/NCT 01323530/2011_03_24 [retrieved on 2017-10-27]
- Anonymous: "NCT01439282 on 2011_09_22: ClinicalTrials.gov Archive - Eribulin in combination with capecitabine for adjuvant treatment in estrogen receptor-positive early stage breast cancer", , 22 September 2011 (2011-09-22), XP055419868, Retrieved from the Internet: URL:https://clinicaltrials.gov/archive/NCT 01439282/2011_09_22 [retrieved on 2017-10-27]
- MUHAMMAD YASER NASIM ET AL: "A phase Ib dose-escalation study of eribulin mesylate in combination with capecitabine in patients with advanced/metastatic cancer", JOURNAL OF CLINICAL ONCOLOGY, vol. 30, no. 15 supp, 31 May 2012 (2012-05-31), XP055419871,
- JW SMITH ET AL: "Eribulin mesylate (Erib) plus capecitabine (X) for adjuvant treatment in post-menopausal estrogen receptor-positive (ER+) early-stage breast cancer: Phase II, multicenter, single-arm study", JOURNAL OF CLINICAL ONCOLOGY, vol. 31, no. 15 supp, 31 May 2013 (2013-05-31), XP055419877,
- 'Phase II Neoadjuvant Trial of Eribulin Followed by Dose Dense Doxorubicin and CycloPhosphamide for Her2-Negative, Locally Advanced Breast Cancer.' NCT01498588 22 December 2011, page 1, XP055361012 Retrieved from the Internet: <URL:ClinicalTrials.gov> [retrieved on 2015-07-22]
- 'A Phase I Dose Escalation Study of Eribulin in Combination With Weekly Carboplatin for the Treatment of Metastatic Breast Cancer.' NCT01795586. 19 February 2013, XP055361013 Retrieved from the Internet: <URL:ClinicalTrials.gov> [retrieved on 2015-07-22]
- 'NCT01388647. Phase I/II Study of Neoadjuvant Carboplatin, Eribulin Mesylate and Trastuzumab (ECH) for Operable HER2 Positive Breast Cancer.' CLINICALTRIALS.GOV 06 July 2011, page 1, XP055361014 Retrieved from the Internet: <URL:https://clinicaltrials.gov/archive/NCT 01388647/2011_07_06> [retrieved on 2015-07-22]
- 'NCT01439282. A Phase II' MULTICENTER, SINGLE.ARM, FEASIBILITY STUDY OF ERIBULIN IN COMBINATION WITH CAPECITABINE FOR ADJUVANT TREATMENT IN ESTROGEN RECEPTO.?POSITIVEEARLY STAGE BREAST CANCER. 22 September 2011, page 1, XP055361015 Retrieved from the Internet: <URL:ClinicalTrials.gov> [retrieved on 2015-07-22]
- ANDRE, F ET AL.: 'Optimal Strategies for the Treatment of Metastatic Triple-Negative Brest Cancer with Currently Approved Agents.' ANNALS OF ONCOLOGY. vol. 23, no. 6, 2012, ISSN 0923-7534 pages VI46 - VI51, XP055098263
- JORDAN, MA: 'Mechanism of Action of Antitumor Drugs That Interact with Microtubules and Tubulin.' CURRENT MEDICINAL CHEMISTRY - ANTI-CANCER AGENTS. vol. 2, no. 1, 2002, pages 1 - 17, XP009101431

## Description

### BACKGROUND OF THE INVENTION

Cancer is a term used to describe a wide variety of diseases that are each characterized by the uncontrolled growth of a particular type of cell. It begins in a tissue containing such a cell and, if the cancer has not spread to any additional tissues at the time of diagnosis, may be treated by, for example, surgery, radiation, or another type of localized therapy. However, when there is evidence that cancer has metastasized from its tissue of origin, different approaches to treatment are typically used. Indeed, because it is not possible to determine with certainty the extent of metastasis, systemic approaches to therapy are usually undertaken when any evidence of spread is detected. These approaches can involve the administration of chemotherapeutic drugs that interfere with the growth of rapidly dividing cells, such as cancer cells. Other approaches involve the use of immunotherapy, in which an immune response against cancerous cells in a subject is elicited or enhanced.

Halichondrin B is a structurally complex, macrocyclic compound that was originally isolated from the marine sponge *Halichondria okadai,* and subsequently was found in *Axinella sp., Phakellia carteri,* and *Lissodendoryx sp.* A total synthesis of halichondrin B was published in 1992 (Aicher et al., J. Am. Chem. Soc. 114:3162-3164, 1992). Halichondrin B has been shown to inhibit tubulin polymerization, microtubule assembly, betas-tubulin crosslinking, GTP and vinblastine binding to tubulin, and tubulin-dependent GTP hydrolysis *in vitro.* This molecule has also been shown to have anti-cancer properties *in vitro* and *in vivo.* Halichondrin B analogs having anti-cancer activities are described in U.S. Patent No. 6,214,865 B1. US6653341 and US2006/104984 both describe halichondrin B in combination with other chemotherapeutic agents. Various clinical trials have also been conducted to assess therapeutic combinations including eribulin with capecitabine (NCT01323530, NCT01439282, Nasim et al., J Clin Oncol, 30:2552-2552, 2012 and Smith et al., J Clin Oncol 31:563-563, 2013) and eribulin in combination with doxorubicin and cylophosphamide (NCT01498588) and carboplatin (NCT01795586).

Eribulin is a synthetic analog of halichondrin B. Eribulin is also known as ER-086526, and has been assigned CAS number 253128-41-5 and US NCI designation number NSC-707389. The mesylate salt of eribulin (eribulin mesylate, which is marketed under the trade name HALAVEN® and is also known as E7389) received FDA approval in November of 2010 for the treatment of patients with metastatic breast cancer who have previously received at least two chemotherapeutic regimens for the treatment of metastatic disease that should have included an anthracycline and a taxane in either the adjuvant or metastatic setting.

The chemical name for eribulin mesylate is 11,15:18,21:24,28-triepoxy-7,9-ethano-12,15-methano-9*H*,15*H*-furo[3,2-*i*]furo[2',3':5,6]pyrano[4,3-b][l,4]dioxacyclopentacosin-5(4*H*)-one, 2-[(2S)-3-amino-2-hydroxypropyl]hexacosahydro-3-methoxy-26-methyl-20,27-bis(methylene)-, (2*R*,3*R*,3a*S*,7*R*,8a*S*,9*S*,10a*R*,11*S*,12*R*,13a*R*,13b*S*,15*S*,18*S*,21*S*,24*S*,26*R*,28*R*,29a*S*)-methanesulfonate (salt), and it can be depicted as follows:

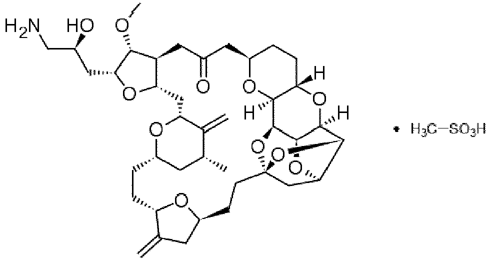

### SUMMARY OF THE INVENTION

The invention is defined by the claims. Any subject-matter falling outside the scope of the claims is provided for information purposes only.

The invention is based in part on observations that administration of eribulin mesylate prior to certain other agents (such as capecitabine, paclitaxel, and carboplatin), shows improved (e.g., synergistic) antitumor effects. Also disclosed are methods of preventing and treating cancer by administration of eribulin or a pharmaceutically acceptable salt thereof (e.g., eribulin mesylate), followed by one or more other agents [e.g., capecitabine, an anti-mitotic agent (e.g., paclitaxel or docetaxel), a platinum-based antineoplastic agent (e.g., carboplatin, cisplatin, or oxaliplatin), doxorubicin, or ixabepilone].

When the term "eribulin" is used herein, it should be considered as indicating eribulin or a pharmaceutically acceptable salt thereof (such as eribulin mesylate), unless the context indicates otherwise.

In a first aspect, the invention provides eribulin or a pharmaceutically acceptable salt thereof for use in a method for treating a subject having or at risk of developing breast cancer, the method comprising administering to the subject (i) eribulin or a pharmaceutically acceptable salt thereof and, subsequently, (ii) a second agent which is capecitabine, wherein eribulin or a pharmaceutically acceptable salt thereof is administered 1, 2, 3, 4, 5 or 6 days or 1-12 weeks before the second agent. Also disclosed is eribulin or a pharmaceutically acceptable salt thereof for use in methods for treating a subject (e.g., a human patient) having or at risk of developing breast cancer (e.g., a subject diagnosed with breast cancer, in treatment for breast cancer, or in post-therapy recovery from breast cancer). The methods involve administering to the subject (i) eribulin or a pharmaceutically acceptable salt thereof (e.g., eribulin mesylate) and, subsequently, (ii) a second agent selected from the group consisting of capecitabine, an anti-mitotic agent (e.g., paclitaxel or docetaxel), a platinum-based anti-neoplastic agent (e.g., cisplatin, carboplatin, or oxaliplatin), doxorubin, and ixabepilone. The treatment can optionally be carried out as neoadjuvant treatment prior to, e.g., surgery. In various examples, the breast cancer is a primary tumor, locally advanced, metastatic, and/or characterized as estrogen receptor positive or negative, progesterone receptor positive or negative, HER-2 positive or negative, or triple-negative breast cancer.

In certain embodiments, the eribulin or the pharmaceutically acceptable salt thereof (e.g., eribulin mesylate) is administered by intravenous infusion (e.g., for about 1 to about 20 minutes, or about 2 to about 5 minutes), in an amount in the range of about 0.1 mg/m² to about 20 mg/m² (e.g., about 1.1 mg/m² or 1.4 mg/m²), and/or once on each of days 1 and 8 of a 21-day cycle.

In various embodiments, the eribulin or the pharmaceutically acceptable salt thereof (e.g., eribulin mesylate) is completed prior to administration of the second agent, while in other embodiments, administration of the eribulin or the pharmaceutically acceptable salt thereof (e.g., eribulin mesylate) continues after administration of the second agent begins.

The second agent administered is capecitabine, which can, for example, be administered daily for two weeks, followed by a one-week rest.

In various embodiments, erublin or a pharmaceutically acceptable salt thereof for use in a method of treatment according to the invention: (i) reduces the number of cancer cells; (ii) reduces tumor volume; (iii) increases tumor regression rate; (iv) reduces or slows cancer cell infiltration into peripheral organs; (v) reduces or slows tumor metastasis; (vi) reduces or inhibits tumor growth; (vii) prevents or delays occurrence and/or recurrence of the cancer and/or extends disease- or tumor-free survival time; (viii) increases overall survival time; (ix) reduces the frequency of treatment; and/or (x) relieves one or more of symptoms associated with the cancer.

In another aspect, the invention also includes eribulin or a pharmaceutically acceptable salt thereof for use in methods for decreasing the size of a tumor in a subject having breast cancer. These methods include administering to the subject (i) eribulin or a pharmaceutically acceptable salt thereof (e.g., eribulin mesylate) and, subsequently, (ii) a second agent: which is capecitabine. The eribulin or pharmaceutically acceptable salt thereof (e.g., eribulin mesylate) and/or other agent can be administered, optionally, as described above and elsewhere herein, and can result in, e.g., one or more of the effects listed above (i-x). This treatment can optionally be carried out in the neoadjuvant context prior to, e.g., surgery. In various examples, the breast cancer is a primary tumor, locally advanced, metastatic, and/or characterized as estrogen receptor positive or negative, progesterone receptor positive or negative, HER-2 positive or negative, or triple-negative breast cancer.

In a further aspect, the disclosure includes increasing the efficacy of an agent selected from the group consisting of capecitabine, an anti-mitotic agent (e.g., paclitaxel or docetaxel), a platinum-based anti-neoplastic agent (e.g., cisplatin, carboplatin, or oxaliplatin), doxorubin, and ixabepilone in treating breast cancer in a subject. These methods involve administering eribulin or a pharmaceutically acceptable salt thereof (e.g., eribulin mesylate) to the subject prior to the agent. In various examples, the eribulin or pharmaceutically acceptable salt thereof (e.g., eribulin mesylate) and/or other agent are administered in schedules and doses, as well as to particular subjects, as described above and elsewhere herein (to achieve, e.g., one or more of the effects listed above).

In an additional aspect, the disclosure includes kits for use in treating breast cancer or decreasing tumor size in a subject having breast cancer. The kits include (i) eribulin or a pharmaceutically acceptable salt thereof (e.g., eribulin mesylate), and (ii) a second agent selected from the group consisting of capecitabine, an anti-mitotic agent (e.g., paclitaxel or docetaxel), a platinum-based anti-neoplastic agent (e.g., cisplatin, carboplatin, or oxaliplatin), doxorubin, and ixabepilone, optionally in dosage form. Optionally, the kits include instructions for use and/or devices or reagents for administration.

The disclosure also includes the use of eribulin, or a pharmaceutically-acceptable salt thereof (e.g., eribulin mesylate), and a subsequently administered second agent, as described herein, for treating a subject having or at risk of developing breast cancer, as described herein, or for preparing a medicament for use in this purpose.

The methods and uses of the disclosure provide improved efficacy against cancer. Combination methods in some instances can, for example, be used to obtain synergistic effects in which, for example, the effects are greater than the sum of the effects of drugs administered individually. Combination methods that result in additive effects are also beneficial.

Other features and advantages of the disclosure will be apparent from the following detailed description, drawings, and claims.

### BRIEF DESCRIPTION OF THE DRAWINGS

Fig. 1 is a graph showing the antitumor effect of capecitabine after eribulin treatment in a human breast cancer cell line MDA-MB-231 s.c. xenograft model.
Fig. 2 is a graph showing the antitumor effect of sequential treatment of eribulin and paclitaxel (PTX) in a human breast cancer cell line MDA-MB-231 s.c. xenograft model.
Figs. 3A and 3B are graphs showing the antitumor effect of sequential treatment of eribulin plus carboplatin (CBDCA) and paclitaxel plus carboplatin in a human breast cancer cell line MDA-MB-231 s.c. xenograft model. Fig. 3A shows anti-tumor effect and Fig. 3B shows body weight of sequential treatment.
Fig. 4 is a graph showing the concentration of carboplatin in MDA-MB-231 tumors after treatment with eribulin.

### DETAILED DESCRIPTION

The disclosure provides eribulin or a pharmaceutically acceptable salt thereof for use in methods for the prevention and treatment of cancer involving administration of eribulin or a pharmaceutically acceptable salt thereof (e.g., eribulin mesylate) prior to a second (or further) anticancer agent such as, for example, capecitabine, an anti-mitotic agent (e.g., paclitaxel or docetaxel), a platinum-based antineoplastic agent (e.g., carboplatin, cisplatin, or oxaliplatin), doxorubicin, or ixabepilone. Eribulin or a pharmaceutically acceptable salt thereof may be administered prior to a second anticancer agent used in the first, second, or third line in the treatment of breast cancer, in the neoadjuvant setting, to enhance the efficacy of the second anticancer agent.

Treatment of cancer using eribulin or a pharmaceutically acceptable salt thereof according to the invention, can (i) reduce the number of cancer cells; (ii) reduce tumor volume; (iii) increase tumor regression rate; (iv) reduce or slow cancer cell infiltration into peripheral organs; (v) reduce or slow tumor metastasis; (vi) reduce or inhibit tumor growth; (vii) prevent or delay occurrence and/or recurrence of the cancer and/or extend disease- or tumor-free survival time; (viii) increase overall survival time; (ix) reduce the frequency of treatment; and/or (x) relieve one or more of symptoms associated with the cancer.

### Pharmaceutical Compositions. Dosage, and Methods

Pharmaceutical compositions including eribulin and/or the other agents described herein can be prepared using standard methods known in the art, or can be obtained from commercial sources. Typically, eribulin and the other agents used in the invention are included within separate pharmaceutical compositions but they can, optionally, be included within a single composition. Eribulin, anti-mitotic agents (e.g., paclitaxel or docetaxel), platinum-based anti-neoplastic agents (e.g., carboplatin, cisplatin, and oxaliplatin), doxorubicin, and ixabepilone are typically provided in liquid form, for intravenous administration, while capecitabine is typically provided in tablet form, for oral administration.

Pharmaceutical compositions used in the invention can be prepared by, for example, mixing or dissolving the active ingredient(s), having the desired degree of purity, in a physiologically acceptable diluent, carrier, excipient, or stabilizer (see, e.g., Remington's Pharmaceutical Sciences (20th edition), ed. A. Gennaro, 2000, Lippincott, Williams & Wilkins, Philadelphia, PA). Acceptable diluents include water and saline, optionally including buffers such as phosphate, citrate, or other organic acids; antioxidants including butylated hydroxytoluene (BHT), butylated hydroxyanisole (BHA), ascorbic acid; low molecular weight (less than about 10 residues) polypeptides; proteins, such as serum albumin, gelatin or immunoglobulins; hydrophilic polymers such as polyvinylpyrrolidone, amino acids such as glycine, glutamine, asparagines, arginine or lysine; monosaccharides, disaccharides, or other carbohydrates including glucose, mannose, or dextrins; chelating agents such as EDTA; sugar alcohols such as mannitol or sorbitol; salt-forming counterions such as sodium; and/or nonionic surfactants such as TWEEN™, PLURONICS™, or PEG.

In preparing compositions for oral dosage form (e.g., compositions including capecitabine), any of the usual pharmaceutical media can be employed, for example, water, glycols, oils, alcohols, flavoring agents, preservatives, coloring agents. In addition, carriers such as starches, sugars, microcristalline cellulose, diluents, granulating agents, lubricants, binders, disintegrating agents, and the like can be used in the case of oral solid preparations such as, for example, powders, capsules, and tablets.

Optionally, the formulations of the invention contain a pharmaceutically acceptable preservative. In some embodiments the preservative concentration ranges from 0.1 to 2.0%, typically v/v. Suitable preservatives include those known in the pharmaceutical arts, such as benzyl alcohol, phenol, m-cresol, methylparaben, and propylparaben. Further, the eribulin and/or other drug formulations can optionally include a pharmaceutically acceptable salt, such as sodium chloride at, for example, about physiological concentrations. Thus, in one example, eribulin (e.g., eribulin mesylate) is formulated in 0.9% Sodium Chloride Injection (USP).

The formulations noted above (and others) can be used for parenteral administration of the drugs. Thus, the drugs can be administered by routes including intravenous, intra-tumoral, peri-tumoral, intra-arterial, intra-dermal, intra-vesical, ophthalmic, intramuscular, intradermal, intraperitoneal, pulmonary, subcutaneous, and transcutaneous routes. Other routes can also be used including, for example, transmucosal, transdermal, inhalation, intravaginal, rectal, and oral administration routes.

The dosage of eribulin and the other agents described herein administered can differ markedly depending on the type of target disease, the choice of delivery method, as well as the age, sex, and weight of the patient, the severity of the symptoms, along with other factors.

Each of the drugs used in the methods is described below, in addition to regimens of administration. These descriptions are followed by examples of how eribulin pre-treatment can be carried out according to the combination methods.

### Eribulin

Methods for the synthesis of eribulin are described, for example, in U.S. Patent No. 6,214,865; U.S. Patent No. 7,982,060; U.S. Patent No. 8,350,067; and U.S. Patent No. 8,093,410. As noted above, eribulin mesylate is available commercially and is marketed as HALAVEN®.

As noted above, eribulin can optionally be used in the present invention in salt forms. There are no particular limitations as to the salt used, whether inorganic acid salt or organic acid salt. For example, the salt can be selected from mesylic acid salt (e.g., eribulin mesylate), hydrochloric acid salt, sulfuric acid salt, citrate, hydrobromic acid salt, hydroiodine acid salt, nitric acid salt, bisulfate, phosphoric acid salt, super phosphoric acid salt, isonicotinic acid salt, acetic acid salt, lactic acid salt, salicic acid salt, tartaric acid salt, pantotenic acid salt, ascorbic acid salt, succinic acid salt, maleic acid salt, fumaric acid salt, gluconic acid salt, saccharinic acid salt, formic acid salt, benzoic acid salt, glutaminic acid salt, methanesulfonic acid salt, ethanesulfonic acid salt, benzenesulfonic acid salt, p-toluenesulfonic acid salt, pamoic acid salt (pamoate), and so on. Moreover, it is acceptable to use salt of aluminum, calcium, lithium, magnesium, sodium, zinc, and diethanolamine.

The daily dosage of eribulin (e.g., eribulin mesylate) can be in the range of, e.g., 0.001 mg/m² to about 100 mg/m² (e.g., in the range of about 0.1 mg/m² to about 50 mg/m² or in the range of about 0.7 mg/m² to about 1.5 mg/m², or in any single amount within these ranges (e.g., 1.4 mg/m² or 1.1 mg/m²)). Eribulin can be administered as a single dose once a day, week, month, or year, or more than one dose of eribulin can be administered per day, week, month, or year. For example, in one administration protocol, eribulin can be administered once on days 1 and 8 of a 21-day cycle. More specifically, a recommended dose of eribulin mesylate is 1.4 mg/m² administered intravenously over 2 to 5 minutes on days 1 and 8 of a 21-day cycle. A recommended dose of eribulin mesylate in patients with mild hepatic impairment (Child-Pugh A) is 1.1 mg/m² administered intravenously over 2 to 5 minutes on days 1 and 8 of a 21-day cycle, while a recommended dose of eribulin mesylate in patients with moderate hepatic impairment (Child-Pugh B) is 0.7 mg/m² administered intravenously over 2 to 5 minutes on days 1 and 8 of a 21-day cycle. Further, a recommended dose of eribulin mesylate in patients with moderate renal impairment (creatinine clearance of 30-50 mL/min) is 1.1 mg/m² administered intravenously over 2 to 5 minutes on days 1 and 8 of a 21-day cycle. These or other lower doses of eribulin mesylate can optionally be used in the context of combination treatment, according to the invention.

### Capecitabine

Capecitabine (XELODA, Roche), a fluoropyrimidine carbamate, is a prodrug of 5'-deoxy-5-fluorouridine (5'-DFUR). The daily dosage of capecitabine is not particularly restricted, although the drug can typically be administered in the range of 1000-4000 mg/m² (e.g., 1500-3500 mg/m², 2000-3000 mg/m², or 2500 mg/m²). It can be administered as a single dose once a day, week, month, or year, or more than one dose (e.g., 2 doses) of capecitabine can be administered per day, week, month, or year. For example, in one administration protocol, 2500 mg/m² capecitabine is administered orally daily (in two divided doses) for two weeks, followed by a 1-week rest period, thus forming a 3 week cycle. In various embodiments, the amount of capecitabine may be reduced, as compared to standard doses, in view of the co-administration of eribulin (e.g., eribulin mesylate).

### Anti-mitotic agents

### Paclitaxel

Paclitaxel (TAXOL®, Bristol-Myers Squibb) is a mitotic inhibitor. The daily dosage of paclitaxel is not particularly restricted, although the drug can typically be administered in the range of 50-300 mg/m² (e.g., 100-250 mg/m², 150-200 mg/m², or 175 mg/m²). It can be administered as a single dose once a day, week, month, or year, or more than one dose (e.g., 2 doses) of paclitaxel can be administered per day, week, month, or year. For example, in one administration protocol, 135-175 mg/m² paclitaxel is administered intravenously over 1-5 (e.g., 3) hours every three weeks, for 2-6 (e.g., 4) courses. In another example, 50-120 mg/m² (e.g., 70-100 mg/m², or 80-90 mg/m²) paclitaxel is administered weekly for, e.g., 6-12 (e.g., 9) weeks. In various embodiments, the amount of paclitaxel may be reduced, as compared to standard doses, in view of the co-administration of eribulin (e.g., eribulin mesylate).

### Docetaxel

Docetaxel (Taxotere; Docefrez) is a mitotic inhibitor. The daily dosage of docetaxel is not particularly restricted, although the drug can typically be administered in the range of 50-300 mg/m² (e.g., 60-250 mg/m², 75-200 mg/m², or 100-150 mg/m²). It can be administered as a single dose once a day, week, month, or year, or more than one dose (e.g., 2 doses) of docetaxel can be administered per day, week, month, or year. For example, in one administration protocol, 60-100 (e.g., 75) mg/m² docetaxel is administered intravenously over 0.5-2 (e.g., 1) hours every three weeks, for 2-6 (e.g., 4) courses. In various embodiments, the amount of docetaxel may be reduced, as compared to standard doses, in view of the co-administration of eribulin (e.g., eribulin mesylate).

### Platinum-based anti-neoplastic agents

Platinum-based antineoplastic drugs (platins) that may be used include, for example, cisplatin, carboplatin, oxaliplatin, satraplatin, picoplatin, nedaplatin, triplatin, and lipolatin. Dosing and administration regimens for these drugs are well known in the art and may readily be adapted for use in the present invention. In various embodiments, the amount of platinum-based antineoplastic drug may be reduced, as compared to standard doses, in view of the co-administration of eribulin (e.g., eribulin mesylate). In the case of carboplatin, for example, reference may be made to Calvert et al., J. Clin. Oncol. 7:1748-1756, 1989, which explains well known approaches to dose determination based on, for example, glomerular filtration rate. As specific examples, a platinum-based antineoplastic drug (e.g., carboplatin) may be administered in the amount of AUC 4/5/6 IV Q3 weeks.

### Doxorubicin

Doxorubicin (Adriamycin; Rubex; Doxil in lipid encapsulated form) is classified as an anthracycline antiobiotic. The daily dosage of doxorubicin is not particularly restricted, although the drug can typically be administered in the range of 40 to 60 mg/m² IV every 21 to 28 days. Alternatively, 60 to 75 mg/m² IV once every 21 days. It can be administered as a single dose once a day, week, month, or year, or more than one dose (e.g., 2 doses) of doxorubicin can be administered per day, week, month, or year. In various embodiments, the amount of doxorubicin may be reduced, as compared to standard doses, in view of the co-administration of eribulin (e.g., eribulin mesylate).

### Ixabepilone

Ixabepilone (IXEMPRA) is a microtubule inhibitor belonging to the epithilone class of antineoplastic agents. Epothilones are isolated from the myxobacterium *Sorangium cellulosum.* Ixabepilone is a semisynthetic analog of epothilone B, a 16-membered polyketide macrolide, with a chemically modified lactam substitution for the naturally existing lactone. The daily dosage of ixabepilone is not particularly restricted, although the drug can typically be administered in the range of 20-60 (e.g., 40) mg/m² intravenously over 1-4 (e.g., 2-3) hours every 3 weeks. It can be administered as a single dose once a day, week, month, or year, or more than one dose (e.g., 2 doses) of ixabepilone can be administered per day, week, month, or year. In various embodiments, the amount of ixabepilone may be reduced, as compared to standard doses, in view of the co-administration of eribulin (e.g., eribulin mesylate).

### Combination administration regimens

As noted above, according to the invention, eribulin (e.g., eribulin mesylate) is administered prior to capecitabine. Thus, for example, eribulin (e.g., eribulin mesylate) can be administered 1, 2, 3, 4, 5, or 6 days, or 1-12 (e.g., 2-10 or 4-8) weeks before capecitabine, as determined to be appropriate by those of skill in the art.

In various examples, one or more (e.g., 1-8, 2-7, 3-6, or 4-5) doses or complete cycles of eribulin treatment can be administered prior to one or more (e.g., 1-8, 2-7, 3-6, or 4-5) doses or complete cycles of treatment with capecitabine described herein. Thus, in one example, eribulin (e.g., eribulin mesylate; e.g., 1.4 mg/m² or 1.1 mg/m² administered over, e.g., 2 to 5 minutes) is administered on days 1 and 8 of a 21-day cycle, and 1-8, 2-7, 3-6, or 4-5 (e.g., 4) complete cycles are carried out. Eribulin treatment then stops and treatment using capecitabine, as described herein, begins. In one example of the disclosure, weekly administration of paclitaxel (e.g., 80 mg/m²) then begins for 1-20 (e.g., 2-18, 3-16, 4-14, 5-12, 6-11, or 7-9) weeks. Thus, in this example, eribulin pre-treatment is completed before paclitaxel administration begins. In a variation of this method, a third agent (e.g., a platinum-based antineoplastic agent, such as carboplatin, cisplatin, or oxaliplatin; e.g., AUC 6) is administered starting on day 1 of eribulin administration and continuing every 3 weeks throughout the course of eribulin and paclitaxel treatment. In other examples, eribulin (e.g., eribulin mesylate; e.g., 1.4 mg/m² or 1.1 mg/m² administered over, e.g., 2 to 5 minutes) is administered on days 1 and 8 of a 21-day cycle, and 1-8, 2-7, 3-6, or 4-5 (e.g., 4) complete cycles are carried out. After the last eribulin cycle, one or more (e.g., 1-8, 2-7, 3-6, or 4-5) cycles of administration of capecitabine, docetaxel, a platinum-based antineoplastic agent (e.g., carboplatin, cisplatin, or oxaliplatin), doxorubicin, or ixabepilone is carried out, as described above in connection with each of these agents.

In other examples, eribulin treatment is started and continues (whether during the course of a cycle or with the start of a cycle) at the same time that treatment using the other agent begins. In one example of this approach, eribulin is administered on day 1 of a 21-day cycle. On day 8 of this cycle, eribulin is administered again and a treatment course using one of the other agents begins. In the case of capecitabine, for example, the administration can be daily for two weeks, and the 1-week rest period noted above can either precede or coincide with the start of another eribulin 21-day cycle. In another example, eribulin is administered on day 1 of a 21-day cycle. On day 8 of this cycle, eribulin is administered again and a second drug (e.g., paclitaxel, docetaxel, cisplatin, carboplatin, oxaliplatin, doxorubicin, or ixabepilone) is administered on this day as well, and then is administered every three weeks, overlapped on further cycles of eribulin administration. Optionally, in the case of eribulin pre-treatment prior to paclitaxel, docetaxel, doxorubicin, or ixabepilone treatment, a platinum-based antineoplastic drug (e.g., cisplatin, carboplatin, oxaliplatin) is administered once during this 21 day cycle. This administration may take place on the first day of the cycle or at any day determined to be appropriate by those of skill in the art. This course of treatment may be repeated, as determined to be tolerable and effective by those of skill in the art.

In addition to eribulin and capecitabine noted above the present invention can also include the administration of one or more additional therapeutic agents. Among these agents, immunomodulatory agents (e.g., antibodies or vaccines), chemotherapeutic/antitumor agents, antibacterial agents, anti-emetics, and anti-inflammatory agents are suitable. Eribulin for use in the methods according to the disclosure can consist of administration of (i) eribulin or a pharmaceutically acceptable salt thereof (e.g., eribulin mesylate), and (ii) capecitabine, an anti-mitotic agent (e.g., paclitaxel or docetaxel), a platinum-based antineoplastic agent (e.g., carboplatin, cisplatin, or oxaliplatin), doxorubicin, or ixabepilone.

Eribulin can be used to treat (including, e.g., delay progression) or prevent cancer in a subject (e.g., a human patient) and/or to decrease tumor size. The subject can be diagnosed with cancer, at risk for developing cancer, in treatment for cancer, or in post-therapy recovery from cancer. Further, eribulin can be used to treat or prevent metastases and/or recurrence. The treatment can be chemotherapeutic alone, although treatment in combination with a surgical procedure to remove or reduce the size of a tumor, radiation therapy, immunotherapy, and/or ablation therapy is also envisioned.

As noted above, eribulin can be used to treat breast cancer. Types of breast cancer that can be treated include, e.g., estrogen receptor positive or negative, progesterone receptor positive or negative, HER-2 positive or negative, or triple-negative breast cancer. The methods can further be used to treat locally advanced or metastatic breast cancer. In various examples, the methods are carried out in the neoadjuvant setting, and thus are carried out before another, primary treatment, such as surgery.

### Kits

Also disclosed are kits that include a container with eribulin (e.g., eribulin mesylate) and/or a container with one or more of the other agents described herein (capecitabine, an anti-mitotic agent (e.g., paclitaxel or docetaxel), a platinum-based antineoplastic agent (e.g., carboplatin, cisplatin, or oxaliplatin), doxorubicin, or ixabepilone. The eribulin and/or other agent in such kits can be provided in amounts sufficient to treat cancer in a patient in need thereof (e.g., amounts sufficient for a single administration or for multiple administrations). The kits can thus include multiple containers that each include effective amounts of single-dose eribulin and/or other agent pharmaceutical composition(s). Optionally, instruments and/or devices necessary for administering the pharmaceutical composition(s) can also be included in the kits. Furthermore, the kits can include additional components, such as instructions or administration schedules, for treating a patient with cancer with the eribulin and/or the other agents described herein.

### Experimental Examples

### Materials and Methods

### Compounds, cell lines, and animals

Eribulin (E7389, Halaven®) and capecitabine were manufactured at Eisai Co., Ltd (Tokyo, Japan), and paclitaxel and carboplatin were purchased from LC Laboratories (Woburn, MA) and BMS (Tokyo, Japan and Sigma-Aldrich, St. Louis, MO), respectively. Human breast cancer cell line, MDA-MB-231, was obtained from ATCC (Rockville, MD). Female athymic nude mice were obtained from Charles River Laboratories JAPAN Inc. (Kanagawa, Japan), Taconic (Germantown, NY), or Japan SLC Co., Ltd. (Shizuoka, Japan). All of the animal experiments were conducted in accordance with the guidelines for animal experiments of Eisai Co., Ltd. or Eisai Inc.

### Preparation of test compound dosing formulations

Eribulin was dissolved in saline at concentration of 0.1 mg/mL. Capecitabine was suspended in 40 mmol/L citrate buffer containing 5% Arabic gum (pH 6.0) to obtain a concentration of 54 mg/mL. Paclitaxel (PTX) was dissolved Cremophore EL:Ethanol=1:1 at concentration of 40 mg/mL and diluted 10 times with 5% glucose solution. Ready-made 10 mg/mL carboplatin solution was used for this assay. The solution was injected at a concentration of 100 mg/kg.

### Antitumor effect of capecitabine after eribulin treatment in human breast cancer cell line MDA-MB-231 s.c. xenograft models

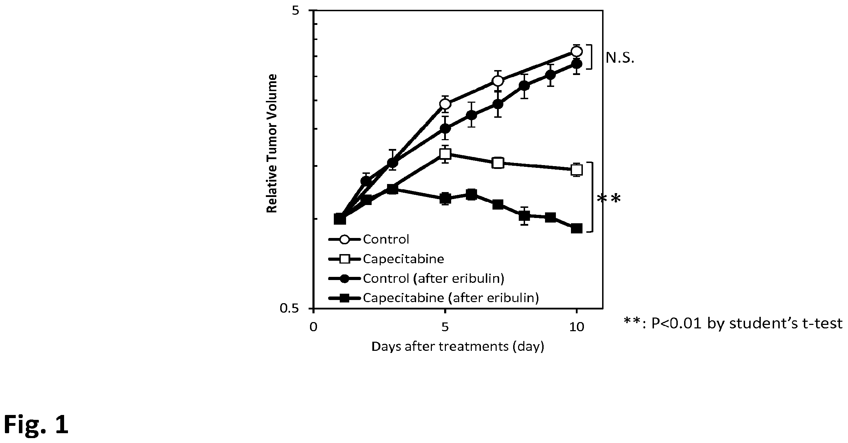

MDA-MB-231 cells were cultured with RPMI 1640 medium containing 10% (v/v) FBS. Cultured tumor cells were suspended in 50% (v/v) BD Matrigel (BD bioscience, San Jose, CA) at a density of 10 × 10⁷ cells/mL for s.c. inoculation into nude mice. A 0.1-mL aliquot of the cell suspension was transplanted subcutaneously into the right flank region of mice. Tumor volumes were calculated according to the following formula: (width [mm]2 × length [mm])/2. Tumor weights were calculated according to the following formula: tumor volume (mm³) × 1 (mg/mm³). In the MDA-MB-231 xenograft model, when the mean tumor volume reached between 100 and 300 mg (1 mm³=1 mg) on day 1, nude mice were randomly divided into each group. Control (no treatment); capecitabine, 540 mg/kg, QD8, p.o.; or eribulin, 1.0 mg/kg, QD1, i.v. was administered on day 1. In the eribulin group, eribulin-treated mice were randomly divided into two groups. On day 12 as a new day 1 in Fig.1 when tumor sizes recovered to initial levels, the two eribulin groups were treated as follows: (i) control after eribulin and (ii) capecitabine, 540 mg/kg, QD8, p.o. after eribulin. The administration volume (0.1 mL/10 g body weight) was calculated based on the body weight before administration. The tumor volume was measured two times per week. The number of mice in each group is 6. Data shown are means +/- SEM (Fig. 1). The data was analyzed by the Dunnett multiple comparison test to compare two groups (GraphPad Prism version 6.02 (GraphPad Software Inc., La Jolla, CA)). A value of *<0.05 was considered statistically significant.

### Antitumor effect of sequential treatment of eribulin and paclitaxel in human breast cancer cell line MDA-MB-231 s.c. xenograft models (reference example)

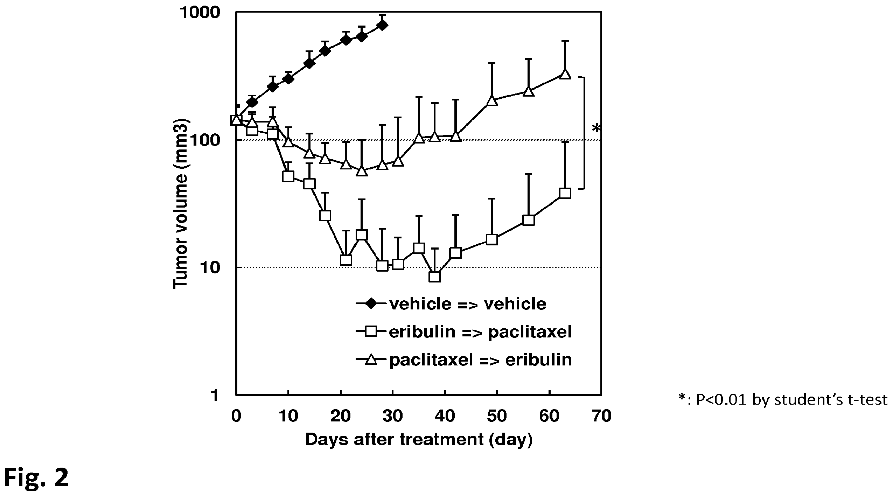

MDA-MB-231 cells were cultured with RPMI 1640 medium containing 10% (v/v) FBS. Cultured tumor cells were suspended in 50% (v/v) GelTrex (Life Technologies, Tokyo, Japan) at a density of 10 × 10⁷ cells/mL for s.c. inoculation into nude mice. A 0.1-mL aliquot of the cell suspension was transplanted subcutaneously into the right flank region of mice. Tumor volumes were calculated according to the following formula: (width [mm]2 × length [mm])/2. Tumor weights were calculated according to the following formula: tumor volume (mm³) × 1 (mg/mm³). In the MDA-MB-231 xenograft model, when the mean tumor volume reached between 100 and 300 mg (1 mm³=1 mg) on day 0, nude mice were randomly divided into each group. Control (no treatment); eribulin, 1 mg/kg i.v.; or PTX, 40 mg/kg i.v. was administered on day 0 and day 7. The administration volume (0.1 mL/10 g body weight) was calculated based on body weight before administration. Tumor volume was measured two times per week. The number of mice in each group is 5. Data shown are means + SD (Fig. 2). The differences in the tumor growth curves between treated groups were analyzed by repeated measures ANOVA. A value of *<0.05(two sided) was considered statistically significant. Statistical analyses were performed using GraphPad Prism version 6.02 (GraphPad Software Inc., La Jolla, CA).

### Antitumor effect of sequential treatment of eribulin plus carboplatin and paclitaxel plus carboplatin in human breast cancer cell line MDA-MB-231 s.c. xenograft (reference example)

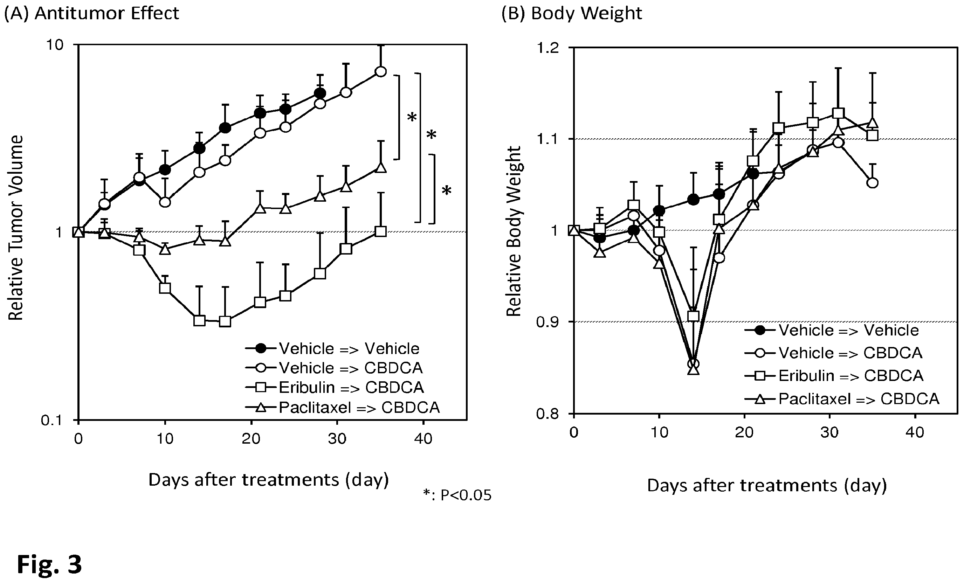

MDA-MB-231 cells were cultured with RPMI 1640 medium containing 10% (v/v) FBS. Cultured tumor cells were suspended in 50% (v/v) GelTrex (Life Technologies, Tokyo, Japan) at a density of 10 × 10⁷ cells/mL for s.c. inoculation into nude mice. A 0.1-mL aliquot of the cell suspension was transplanted subcutaneously into the right flank region of mice. Tumor volumes were calculated according to the following formula: (width [mm]2 × length [mm])/2. Tumor weights were calculated according to the following formula: tumor volume (mm³) × 1 (mg/mm³). In MDA-MB-231 xenograft model, when the mean tumor volume reached between 100 and 300 mg (1 mm³=1 mg) on day 0, nude mice were randomly divided into each group. Control (no treatment); eribulin, 1 mg/kg i.v.; or PTX, 40 mg/kg i.v. was administered on day 0 and carboplatin, 100 mg/kg i.v. was administered on day 7. The administration volume (0.1 mL/10 g body weight) was calculated from the body weight before administration. The tumor volume was measured two times per week. The analysis was performed until on day 35. Number of mice in each group are 5. Data shown are means + SD (Fig. 3). The differences in the tumor growth curves between treated groups were analyzed by repeated measures ANOVA. A value of *<0.05 (two sided) was considered statistically significant. Statistical analyses were performed using GraphPad Prism version 6.02 (GraphPad Software Inc., La Jolla, CA).

### Pharmacokinetics study of carboplatin in tumor after administration of eribulin in human breast cancer cell line MDA-MB-231 s.c. xenograft models (reference example)

Female nude mice, once acclimatised, were subcutaneously injected with 5x10⁶ MDA-MB-231 cells in a 1:1 ratio (50 µL each) with matrigel in a volume of 0.1 mL in phosphate buffered saline, on the right flank area using a 26-gauge needle. This ensured that sufficient mice with tumors in the volume range of approximately 200 ∼ 300 mm³ were available for the study. When the tumors reached an average size of approximately 250 mm³, 30 mice were selected. The study included an initial experiment containing 24 mice. The first group received eribulin mesylate dosed i.v. as a single dose of 3 mg/kg; for a 20 g animal 200 µL of 0.3 mg/mL eribulin mesylate solution was dosed. To control for tumor size, a vehicle control group (vehicle B) consisting of 27 mice, was introduced before carboplatin dosing. The mice in this group were chosen so that their tumor size matches the tumor size of the eribulin mesylate-treated mice group. Vehicle B group was split in 2 groups, first one of 3 mice which received vehicle (saline) i.v. injection and the second group of 24 mice received carboplatin i.v. injection. On day 1 of vehicle B or on day 15 after the treatment of eribulin, 100 mg/kg carboplatin was administered to all mice intravenously and tumor samples were collected under anesthesia at the assigned time points 0.083, 0.25, 0.5, 1, 4, 8, 24, and 48 hours (n=3 for each time point). Tumors were homogenized with 3 times their respective weights of 1 x PBS using a Mini Bead Beater-96, and the concentration of carboplatin in each tumor homogenate was analyzed by a validated LC-MS method.

### Calculation of Pharmacokinetic Parameters

The PK parameters of carboplatin in tumors were calculated using a non-compartmental approach (Pheonix/WinNonlin v. 6.3, Pharsight; Mountain View, CA). The time point at which the concentration of carboplatin highest was denoted as tmax. The highest concentration of carboplatin was denoted as Cmax. The observed area under the tumor concentration versus time curve from time zero to the last quantifiable time point (AUC (0-t)) and to infinity (AUC (0-inf)) were calculated using the trapezoidal method. The AUC (0-inf) was calculated as AUC (0-t) + AUC extrap, where AUC extrap represents the extrapolated AUC from the last quantifiable time point (Clast) to infinity, and was calculated as Clast/λZ. Lamda z (λZ), slope of the concentration versus time curve during the terminal phase, was determined by linear regression.

The mean, standard deviation (SD) and percent coefficient of variation (%CV) of tumor concentrations were calculated using Excel 2010 (Microsoft, Redmond, WA). The mean, SD, and %CV for the PK parameters of carboplatin were determined in WinNonlin or Excel 2010. Due to rounding and formatting differences between WinNonlin and Excel, minor discrepancies in mean, SD, and %CV values might occur. However, these discrepancies were believed insignificant without compromising the integrity of the study.

### Instrumentation and Assay Conditions

### HPLC System

Shimadzu HPLC system (Shimadzu Scientific Instruments, Columbia, MD), which consisted of an autosampler (model: SIL HTc), a column compartment unit (model: CTO-20AC), a degasser (model: DGU-20A3), and a pump (model: LC-20AD), were used.

### MS/MS Conditions

An AB Sciex (Foster City, CA) API5000 mass spectrometer, with electrospray ionization under positive ion mode, was used for sample analysis. The collision-activated dissociation (CAD) with N2 was used to generate structure characteristic product ions for quantitation.

### Calculation of the Concentrations of Carboplatin

The peak areas of carboplatin were integrated using Analyst 1.6 (AB Sciex, Foster City, CA), and the areas were imported into Watson 7.2 (Thermo Fisher Scientific, Waltham, MA). Internal standard, tolbutamide, was added to each sample, excluded blank without IS, however IS was not used for quantitation due to inconsistent instrument response of tolbutamide in all analytical runs. The calibration curves were constructed by plotting peak area of carboplatin against concentrations of calibration standards in tumor homogenate. A 1/x2 weighted linear regression (Y = aX + b) was used to calculate the parameters, a, and b in tumor homogenate. The concentration of carboplatin in each sample was calculated from the regression equations of the respective standard curve. Excel 2010 (Microsoft, Redmond, WA) was used to calculate the mean, SD, percent difference (%Diff), and %CV. All reported data was rounded.

### Results

### Antitumor effect of capecitabine after eribulin treatment in human breast cancer cell line MDA-MB-231 s.c. xenograft models

We evaluated antitumor activity of 540 mg/kg capecitabine in the MDA-MB-231 model with or without pretreatment with 1.0 mg/kg eribulin, to examine whether eribulin pretreatment would alter the drug-sensitivity of tumor cells to a chemotherapeutic agent and to enhance the antitumor activity of capecitabine. Pretreatment of eribulin significantly enhanced the antitumor activity of capecitabine compared with that achieved without pretreatment in MDA-MB-231 xenograft model (Fig. 1).

### Antitumor effect of sequential treatment of eribulin and paclitaxel in human breast cancer cell line MDA-MB-231 s.c. xenograft models

Next, we investigated antitumor activity of sequential treatment of Gr.1; vehicle on day 0 and day 7, Gr. 2; 1 mg/kg eribulin on day 0 and 40 mg/kg paclitaxel on day 7 and Gr. 3; 40 mg/kg paclitaxel on day 0 and 1 mg/kg eribulin on day 7 in the MDA-MB-231 model. Both sequential combination treatments indicated significant antitumor effect and sequential combination treatment of eribulin and paclitaxel showed clear antitumor activities with tumor shrinkage (Fig. 2). The antitumor activity by the treatment of eribulin followed by paclitaxel was significantly stronger than that of paclitaxel followed by eribulin against human breast cancer cell line MDA-MB-231 xenografted in athymic mice.

### Antitumor effect of sequential treatment of eribulin plus carboplatin and paclitaxel plus carboplatin in human breast cancer cell line MDA-MB-231 s.c. xenograft models

Then, we investigated antitumor activity of sequential treatment of 1 mg/kg eribulin on day 0 plus 100 mg/kg carboplatin on day 7 and 40 mg/kg paclitaxel on day 0 plus 100 mg/kg carboplatin on day 7 in the MDA-MB-231 model. Both sequential combination treatments indicated significant antitumor effect and sequential combination treatment of eribulin plus carboplatin showed clear antitumor activities with tumor shrinkage (Fig. 3). The antitumor activity by the treatment of eribulin followed by carboplatin showed significantly stronger than that of paclitaxel followed by carboplatin against human breast cancer cell line MDA-MB-231 xenografted in athymic mice.

### Pharmacokinetics study of carboplatin in tumor after administration of eribulin in human breast cancer cell line MDA-MB-231 s.c. xenograft models

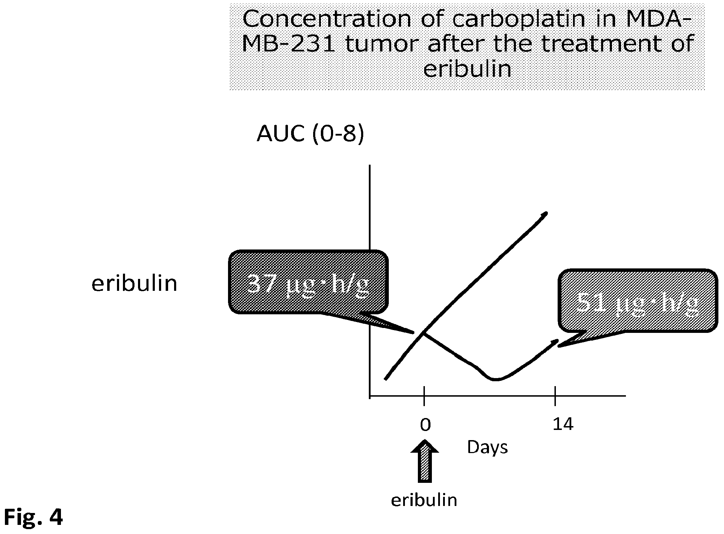

As shown in Table 1, below, and Fig. 4, the eribulin pretreatment group mice had a high exposure of carboplatin in tumors. As noted in Table 1, in the eribulin pretreatment group the Cmax was about 2.20-fold of that of Vehicle B group, and the AUC (0-8) was about 1.37-fold of that of Vehicle B group.

**Table 1. Pharmacokinetics Study of Carboplatin in Tumor after Administration of Eribulin in Human Breast Cancer Cell Line MDA-MB-231 s.c. Xenograft Models**

| **Group** | **Tumor Volume (mm³)** | **Concentration of Carboplatin in MDA-MB-231 Tumor** | |
|---|---|---|---|
| | | Cmax (ng/g) | AUC (0-8) (ng.h/g) |
| Vehicle B | 142.56 | 22540.99 | 36864.98 |
| Eribulin pretreatment | 135.92 | 49677.15 | 50504.02 |

### Other Embodiments

Use of singular forms herein, such as "a" and "the," does not exclude indication of the corresponding plural form, unless the context indicates to the contrary. Similarly, use of plural terms does not exclude indication of a corresponding singular form.

## Claims

1. Eribulin or a pharmaceutically acceptable salt thereof for use in a method for treating a subject having or at risk of developing breast cancer, the method comprising administering to the subject (i) eribulin or a pharmaceutically acceptable salt thereof and, subsequently, (ii) a second agent which is capecitabine, wherein eribulin or a pharmaceutically acceptable salt thereof is administered 1, 2, 3, 4, 5 or 6 days or 1-12 weeks before the second agent.

2. Eribulin or a pharmaceutically acceptable salt thereof for use according to claim 1, wherein said subject is a human patient.

3. Eribulin or a pharmaceutically acceptable salt thereof for use according to claim 1, wherein:
(a) said subject is diagnosed with breast cancer, in treatment for breast cancer, or in post-therapy recovery from breast cancer; or
(b) said treatment is carried out as neoadjuvant treatment prior to surgery.

4. Eribulin or a pharmaceutically acceptable salt thereof for use according to claim 1, wherein:
(a) said breast cancer is a primary tumor;
(b) said breast cancer is locally advanced;
(c) said breast cancer is metastatic; or
(d) said breast cancer is estrogen receptor positive or negative, progesterone receptor positive or negative, HER-2 positive or negative, or triple-negative breast cancer.

5. Eribulin or a pharmaceutically acceptable salt thereof for use according to claim 1, wherein said pharmaceutically acceptable salt of eribulin is eribulin mesylate.

6. Eribulin or a pharmaceutically acceptable salt thereof for use according to claim 1, wherein said eribulin or said pharmaceutically acceptable salt thereof is administered by intravenous infusion.

7. Eribulin or a pharmaceutically acceptable salt thereof for use according to claim 6, wherein said intravenous infusion is for about 1 to about 20 minutes, optionally about 2 to about 5 minutes.

8. Eribulin or a pharmaceutically acceptable salt thereof for use according to claim 1, wherein:
(a) said eribulin or said pharmaceutically acceptable salt thereof is administered in an amount in the range of about 0.1 mg/m² to about 20 mg/m², optionally about 1.1 mg/m² or 1.4 mg/m², or
(b) said eribulin or said pharmaceutically acceptable salt thereof is administered once on each of days 1 and 8 of a 21-day cycle.

9. Eribulin or a pharmaceutically acceptable salt thereof for use according to claim 1, wherein said second agent is administered daily for two weeks, followed by a one-week rest.

10. Eribulin or a pharmaceutically acceptable salt thereof for use according to claim 1, wherein 1-8, 2-7, 3-6, or 4-5 doses or complete cycles of eribulin are administered prior to 1-8, 2-7, 3-6, or 4-5 doses or complete cycles of said second agent.

11. Eribulin or a pharmaceutically acceptable salt thereof for use according to claim 1, wherein said treating: (i) reduces the number of cancer cells; (ii) reduces tumor volume; (iii) increases tumor regression rate; (iv) reduces or slows cancer cell infiltration into peripheral organs; (v) reduces or slows tumor metastasis; (vi) reduces or inhibits tumor growth; (vii) prevents or delays occurrence and/or recurrence of the cancer and/or extends disease- or tumor-free survival time; (viii) increases overall survival time; (ix) reduces the frequency of treatment; and/or (x) relieves one or more of symptoms associated with the cancer.

12. Eribulin or a pharmaceutically acceptable salt thereof for use in a method for decreasing the size of a tumor in a subject having breast cancer, the method comprising administering to the subject (i) eribulin or a pharmaceutically acceptable salt thereof and, subsequently, (ii) a second agent which is capecitabine, wherein eribulin or a pharmaceutically acceptable salt thereof is administered 1, 2, 3, 4, 5 or 6 days or 1-12 weeks before the second agent.

13. Eribulin or a pharmaceutically acceptable salt thereof for use in a method for increasing the efficacy of an agent which is capecitabine in treating breast cancer in a subject, the method comprising administering eribulin or a pharmaceutically acceptable salt thereof to the subject prior to said agent, wherein eribulin or a pharmaceutically acceptable salt thereof is administered 1, 2, 3, 4, 5 or 6 days or 1-12 weeks before capecitabine.

## Patentansprüche

1. Eribulin oder pharmazeutisch annehmbares Salz davon zur Verwendung in einem Verfahren zum Behandeln eines Subjekts, das Brustkrebs hat oder davon gefährdet ist, wobei das Verfahren ein Verabreichen von (i) Eribulin oder einem pharmazeutisch annehmbaren Salz davon und anschließend (ii) einem zweiten Wirkstoff, der Capecitabin ist, an das Subjekt umfasst, wobei Eribulin oder ein pharmazeutisch annehmbares Salz davon 1, 2, 3, 4, 5 oder 6 Tage oder 1-12 Wochen vor dem zweiten Wirkstoff verabreicht wird.

2. Eribulin oder pharmazeutisch annehmbares Salz davon zur Verwendung nach Anspruch 1, wobei das Subjekt ein menschlicher Patient ist.

3. Eribulin oder pharmazeutisch annehmbares Salz davon zur Verwendung nach Anspruch 1, wobei:
(a) bei dem Subjekt Brustkrebs diagnostiziert wurde, es in Behandlung gegen Brustkrebs oder in Genesung nach der Therapie von Brustkrebs ist; oder
(b) die Behandlung als neoadjuvante Behandlung vor der Operation ausgeführt wird.

4. Eribulin oder pharmazeutisch annehmbares Salz davon zur Verwendung nach Anspruch 1, wobei:
(a) der Brustkrebs ein primärer Tumor ist;
(b) der Brustkrebs lokal fortgeschritten ist;
(c) der Brustkrebs metastatisch ist; oder
(d) der Brustkrebs Östrogenrezeptor-positiver oder -negativer, Progesteronrezeptor-positiver oder -negativer, HER-2-positiver oder -negativer oder dreifach negativer Brustkrebs ist.

5. Eribulin oder pharmazeutisch annehmbares Salz davon zur Verwendung nach Anspruch 1, wobei das pharmazeutisch annehmbare Salz von Eribulin Eribulinmesylat ist.

6. Eribulin oder pharmazeutisch annehmbares Salz davon zur Verwendung nach Anspruch 1, wobei das Eribulin oder pharmazeutisch annehmbare Salz davon durch intravenöse Infusion verabreicht wird.

7. Eribulin oder pharmazeutisch annehmbares Salz davon zur Verwendung nach Anspruch 6, wobei die intravenöse Infusion für etwa 1 bis etwa 20 Minuten, optional etwa 2 bis etwa 5 Minuten ist.

8. Eribulin oder pharmazeutisch annehmbares Salz davon zur Verwendung nach Anspruch 1, wobei:
(a) das Eribulin oder pharmazeutisch annehmbare Salz davon in einer Menge im Bereich von etwa 0,1 mg/m² bis etwa 20 mg/m², optional etwa 1,1 mg/m² oder 1,4 mg/m² verabreicht wird, oder
(b) das Eribulin oder das pharmazeutisch annehmbare Salz davon einmal an jedem der Tage 1 und 8 eines 21-Tage-Zyklus verabreicht wird.

9. Eribulin oder pharmazeutisch annehmbares Salz davon zur Verwendung nach Anspruch 1, wobei der zweite Wirkstoff während zwei Wochen täglich verabreicht wird, gefolgt von einer einwöchigen Erholung.

10. Eribulin oder pharmazeutisch annehmbares Salz davon zur Verwendung nach Anspruch 1, wobei 1-8, 2-7, 3-6 oder 4-5 Dosen oder komplette Zyklen vor den 1-8, 2-7, 3-6 oder 4-5 Dosen oder kompletten Zyklen des zweiten Wirkstoffs verabreicht werden.

11. Eribulin oder pharmazeutisch annehmbares Salz davon zur Verwendung nach Anspruch 1, wobei die Behandlung: (i) die Anzahl Krebszellen reduziert; (ii) das Tumorvolumen reduziert; (iii) die Tumorregressionsrate erhöht; (iv) die Krebszellinfiltration in periphere Organe reduziert oder verlangsamt; (v) die Tumormetastase reduziert oder verlangsamt; (vi) das Tumorwachstum reduziert oder hemmt; (vii) das Auftreten und/oder Wiederauftreten des Krebses verhindert oder verzögert und/oder die Krankheits- oder Tumorfreie Überlebensdauer verlängert; (viii) die Gesamtüberlebensdauer verlängert; (ix) die Behandlungshäufigkeit reduziert; und/oder (x) eines oder mehrere mit dem Krebs verbundene Symptome lindert.

12. Eribulin oder pharmazeutisch annehmbares Salz davon zur Verwendung in einem Verfahren zum Verringern der Größe eines Tumors in einem Subjekt, das Brustkrebs hat, wobei das Verfahren ein Verabreichen von (i) Eribulin oder einem pharmazeutisch annehmbaren Salz davon und anschließend (ii) einem zweiten Wirkstoff, der Capecitabin ist, an das Subjekt umfasst, wobei Eribulin oder ein pharmazeutisch annehmbares Salz davon 1, 2, 3, 4, 5 oder 6 Tage oder 1-12 Wochen vor dem zweiten Wirkstoff verabreicht wird.

13. Eribulin oder pharmazeutisch annehmbares Salz davon zur Verwendung in einem Verfahren zum Erhöhen der Wirksamkeit eines Wirkstoffs, der Capecitabin, das Brustkrebs in einem Subjekt behandelt, ist, wobei das Verfahren ein Verabreichen von Eribulin oder einem pharmazeutisch annehmbaren Salz davon an das Subjekt vor dem Wirkstoff umfasst, wobei Eribulin oder ein pharmazeutisch annehmbares Salz davon 1, 2, 3, 4, 5 oder 6 Tage oder 1-12 Wochen vor dem Capecitabin verabreicht wird.

## Revendications

1. Éribuline ou sel pharmaceutiquement acceptable de celle-ci pour l'utilisation dans un procédé pour traiter un sujet atteint d'un cancer du sein ou à risque de développer un cancer du sein, le procédé comprenant l'administration au sujet (i) d'éribuline ou d'un sel pharmaceutiquement acceptable de celle-ci et, ensuite, (ii) d'un second agent, qui est de la capécitabine, dans lequel l'éribuline ou un sel pharmaceutiquement acceptable de celle-ci est administré 1, 2, 3, 4, 5 ou 6 jours ou d'1 à 12 semaines avant le second agent.

2. Éribuline ou sel pharmaceutiquement acceptable de celle-ci pour l'utilisation selon la revendication 1,
dans lequel ledit sujet est un patient humain.

3. Éribuline ou sel pharmaceutiquement acceptable de celle-ci pour l'utilisation selon la revendication 1, dans lequel :
(a) un cancer du sein est diagnostiqué chez ledit sujet, ledit sujet est traité pour le cancer du sein, ou est en convalescence post-thérapie du cancer du sein ; ou
(b) ledit traitement est réalisé sous forme de traitement néoadjuvant avant la chirurgie.

4. Éribuline ou sel pharmaceutiquement acceptable de celle-ci pour l'utilisation selon la revendication 1, dans lequel :
(a) ledit cancer du sein est une tumeur primaire ;
(b) ledit cancer du sein est localement avancé ;
(c) ledit cancer du sein est métastatique ; ou
(d) ledit cancer du sein est un cancer du sein positif ou négatif au récepteur d'oestrogène, positif ou négatif au récepteur de progestérone, HER-2 positif ou négatif, ou triple-négatif.

5. Éribuline ou sel pharmaceutiquement acceptable de celle-ci pour l'utilisation selon la revendication 1, dans lequel ledit sel pharmaceutiquement acceptable d'éribuline est du mésylate d'éribuline.

6. Éribuline ou sel pharmaceutiquement acceptable de celle-ci pour l'utilisation selon la revendication 1, dans lequel ladite éribuline ou ledit sel pharmaceutiquement acceptable de celle-ci est administré par perfusion intraveineuse.

7. Éribuline ou sel pharmaceutiquement acceptable de celle-ci pour l'utilisation selon la revendication 6, dans lequel ladite perfusion intraveineuse est pendant environ 1 à environ 20 minutes, optionnellement environ 2 à environ 5 minutes.

8. Éribuline ou sel pharmaceutiquement acceptable de celle-ci pour l'utilisation selon la revendication 1, dans lequel :
(a) ladite éribuline ou ledit sel pharmaceutiquement acceptable de celle-ci est administré en une quantité dans la plage d'environ 0,1 mg/m² à environ 20 mg/m², optionnellement d'environ 1,1 mg/m² ou de 1,4 mg/m², ou
(b) ladite éribuline ou ledit sel pharmaceutiquement acceptable de celle-ci est administré une fois, à chacun des jours 1 et 8 d'un cycle de 21 jours.

9. Éribuline ou sel pharmaceutiquement acceptable de celle-ci pour l'utilisation selon la revendication 1, dans lequel ledit second agent est administré tous les jours pendant deux semaines, suivi par un repos d'une semaine.

10. Éribuline ou sel pharmaceutiquement acceptable de celle-ci pour l'utilisation selon la revendication 1, dans lequel d'1 à 8, de 2 à 7, de 3 à 6, ou de 4 à 5 doses ou des cycles complets d'éribuline sont administrés avant d'1 à 8, de 2 à 7, de 3 à 6, ou de 4 à 5 doses ou cycles complets dudit second agent.

11. Éribuline ou sel pharmaceutiquement acceptable de celle-ci pour l'utilisation selon la revendication 1, dans lequel le traitement : (i) réduit le nombre de cellules cancéreuses ; (ii) réduit le volume tumoral ; (iii) augmente le taux de régression tumorale ; (iv) réduit ou ralentit l'infiltration de cellules cancéreuses dans des organes périphériques ; (v) réduit ou ralentit la métastase tumorale ; (vi) réduit ou empêche la croissance tumorale ; (vii) empêche ou retarde la survenue et/ou la récurrence du cancer et/ou prolonge le temps de survie sans maladie ou sans tumeur ; (viii) augmente le temps de survie total ; (ix) réduit la fréquence de traitement ; et/ou (x) réduit un ou plusieurs de symptômes associés au cancer.

12. Éribuline ou sel pharmaceutiquement acceptable de celle-ci pour l'utilisation dans un procédé pour réduire la taille d'une tumeur chez un sujet atteint d'un cancer du sein, le procédé comprenant l'administration au sujet (i) d'éribuline ou d'un sel pharmaceutiquement acceptable de celle-ci et, ensuite, (ii) d'un second agent qui est de la capécitabine, dans lequel l'éribuline ou un sel pharmaceutiquement acceptable de celle-ci est administré 1, 2, 3, 4, 5 ou 6 jours ou d'1 à 12 semaines avant le second agent.

13. Éribuline ou sel pharmaceutiquement acceptable de celle-ci pour l'utilisation dans un procédé pour augmenter l'efficacité d'un agent, qui est de la capécitabine, dans le traitement de cancer du sein chez un sujet, le procédé comprenant l'administration d'éribuline ou d'un sel pharmaceutiquement acceptable de celle-ci au sujet avant ledit agent, dans lequel l'éribuline ou un sel pharmaceutiquement acceptable de celle-ci est administré 1, 2, 3, 4, 5 ou 6 jours ou d'1 à 12 semaines avant la capécitabine.
